(19) Europäisches Patentamt
European Patent Office
Office européen des brevets

(11) EP 0 842 656 A2

(12) **EUROPÄISCHE PATENTANMELDUNG**

(43) Veröffentlichungstag:
**20.05.1998 Patentblatt 1998/21**

(51) Int. Cl.$^6$: **A61K 7/48**, A61K 7/06

(21) Anmeldenummer: **97120279.1**

(22) Anmeldetag: **19.11.1997**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **19.11.1996 DE 19647858**

(71) Anmelder:
**BASF AKTIENGESELLSCHAFT**
**67056 Ludwigshafen (DE)**

(72) Erfinder:
- **Friedrich, Holger, Dr.**
  **67240 Bobenheim-Roxheim (DE)**
- **Hössel, Peter, Dr.**
  **67105 Schifferstadt (DE)**

(74) Vertreter:
**Isenbruck, Günter, Dr.**
**Patent- und Rechtsanwälte Bardehle-Pagenberg-Dost-Altenburg-Frohwitter-Geissler & Partner**
**Theodor-Heuss-Anlage 12**
**68165 Mannheim (DE)**

(54) **Verwendung von Carboxyamid-Polysiloxanen in kosmetischen Formulierungen**

(57) Die Erfindung betrifft die Verwendung von Carboxyamid-Polysiloxanen in kosmetischen Formulierungen, insbesondere in Haut-, Haar-, Nagel- oder Mundpflegemitteln. Der kosmetische Zustand wird verbessert - Naßkämmbarkeit, elektrostatische Aufladung, Griff des trockenen Haares beziehungsweise Verbesserung des Hautgefühls.

EP 0 842 656 A2

**Beschreibung**

Die Erfindung betrifft die Verwendung von Carboxyamid-Polysiloxanen der allgemeinen Formel IV

$$[R_a\text{-}\overset{\displaystyle B\atop |}{Si}O_{(3-a)/2}]_n\text{-}[\overset{\displaystyle R'_b\atop |}{Si}O_{(4-b)/2}]_m\text{-}[\overset{\displaystyle R''_3\atop |}{Si}O_{1/2}]_k \qquad \text{(IV)}$$

wobei R, R'und R" jeweils unabhängig voneiander ausgewählt sind aus $C_{1-6}$-Alkyl oder Phenyl, der Carboxyamid-Polysiloxanen der allgemeinen Formel IVa

$$[R_a\text{-}\overset{\displaystyle B\atop |}{Si}O_{(3-a)/2}]_n\text{-}[\overset{\displaystyle R'_b\atop |}{Si}O_{(4-b)/2}]_m\text{-}[\overset{\displaystyle R''_2R'''\atop |}{Si}O_{1/2}]_k \qquad \text{(IVa)}$$

wobei R' und R" unabhängig voneinander ausgewählt sind aus $C_{1-6}$-Alkyl oder Phenyl, R und R''' unabhängig ausgewählt sind aus $C_{1-6}$-Alkyl, $C_{1-6}$-Alkoxy, OH oder Phenyl, und die Zahl der Arten der durch

$$-\ [\overset{\displaystyle R'_b\atop |}{Si}O_{(4-b)/2}]_m$$

definierten strukturellen Einheiten mindestens 1 beträgt und a aus dem Bereich $0 \leq a \leq 2$ und b aus dem Bereich $1 \leq b \leq 3$ ausgewählt ist, und die durch die Variablen m, n, k festgelegte Zahl der Wiederholungen der Arten von strukturellen Einheiten aus den Bereichen

$1 \leq n \leq 100$
$10 \leq m \leq 800$ und
$0 \leq k \leq \{(2\text{-}b)m + [(1\text{-}a)n + 2]\}$ ausgewählt ist,

sowie B einen organischen Rest der allgemeinen Formel V darstellt,

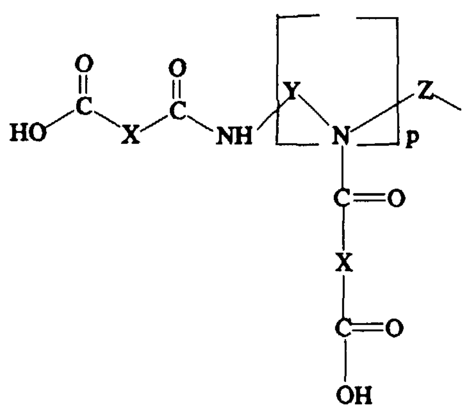

(V)

wobei p = 0 - 10 und

Y und Z unabhängig voneinander jeweils einen divalenten Kohlenwasserstoffrest aus der Gruppe der aliphatischen Kohlenwasserstoffe oder einen divalenten Alkoxyalkylrest darstellen, und

X ein divalenter Kohlenwasserstoffrest aus der Gruppe von $-(CH_2)_y-$ mit $2 \leq y \leq 6$, $-CH_2-CHR^5-$ oder $-CHR^5-CH_2-$ mit $R^5$ $C_{1-20}$-Alkyl oder $C_{2-20}$-Alkenyl, -CH=CH-, cyclischen oder bicyclischen gesättigten oder ungesättigten Kohlenwasserstoffen oder der aromatischen Kohlenwasserstoffe darstellt.

Die konditionierenden und pflegenden Eigenschaften von Marktprodukten, speziell von Siliconölen, von Siliconcopolymeren (z.B. Polysiloxan-Polyether-Copolymere), von Polysiloxan-Polyorganobetain-Copolymeren, von kationisch modifizierten Siliconderivaten (z.B. polyquaternäre Polysiloxan-Polymere, siehe auch DE-A 33 40 708) sind nicht ausreichend und sind verbesserungsbedürftig. Betroffen sind Haar- und Hautpflegeprodukte, z.B. Emulsionen. Am deutlichsten zeigt sich dieses Defizit an der pflegenden Wirkung beim Haarbleichen. Das Haarbleichen schädigt die Haare enorm und bewirkt eine rauhe Oberfläche. Die bisher bekannten Wirkstoffe tragen nur ungenügend zur Pflege und zur Reparatur dieser geschädigten Haare bei.

Aus EP-B 0 579 455 sind Öl-in-Wasser-Emulsionen bekannt, die Wasser, ein Siliconöl und spezifische Emulgatoren enthalten. Diese Emulsionen werden bei kosmetischen Applikationen eingesetzt.

Aus US 5,194,251 ist bekannt, Aminosiloxane, die durch Umsetzung von Polydimethylsiloxanen einer Viskosität von 10 bis 15.000 cs mit bestimmten aminogruppenhaltigen Silanen erhalten werden, bei der Behandlung von Haaren einzusetzen.

Bei der Hydrophobierung von Leder finden allgemein Siliconöle und funktionelle Siloxane Anwendung. Dabei verwendet man bevorzugt carboxylgruppenhaltige Polysiloxane. Derartige carboxylgruppenhaltige Polysiloxane sind in der DE-A 35 29 869, DE-A 38 00 629 sowie WO 95/22627 beschrieben. Die Gebrauchseigenschaften und die anwendungstechnischen Ergebnisse derartiger Siliconölemulsionen sind jedoch noch nicht optimal.

Aus der DE-A 42 14 150 ist ein Verfahren zur Hydrophobierung von Materialien faseriger Struktur bekannt, bei dem Sulfobernsteinsäureester von reaktiven Siloxanen verwendet werden.

Aus der EP-A 0 095 676 sowie Polymer Bulletin 32, Seiten 173-178 (1994) ist die Überführung von Aminoalkylsiloxanen in sogenannten Carboxyamid-Polysiloxane bekannt. Derartige Carboxyamid-Polysiloxane werden auch als "carboxyfunktionalisierte Aminoalkylsiloxane" bezeichnet. Gemäß der EP-A 0 095 676 werden derartige Carboxyamid-Polysiloxane für Textilien und Gewebe verwendet. Sie verleihen diesen Stoffen eine gewisse Weichheit sowie ein gewisses Wasserabstoßungsvermögen. Außerdem können diese Verbindungen auch als Trenn- oder Gleitmittel für metallische Substrate eingesetzt werden.

Angesichts des vorstehend Gesagten ist es Aufgabe der Erfindung, kosmetische Emulsionen bereitzustellen, die die genannten Nachteile, insbesondere die Haarschädigung vermeiden.

Diese Aufgabe wird durch Verwendung von Carboxyamid-Polysiloxanen der allgemeinen Formel IV

$$[R_a\text{-}\underset{\displaystyle B}{\overset{|}{Si}}O_{(3-a)/2}]_n\text{-}[\underset{\displaystyle R'_b}{\overset{|}{Si}}O_{(4-b)/2}]_m\text{-}[\underset{\displaystyle R''_3}{\overset{|}{Si}}O_{1/2}]_k \qquad (IV)$$

wobei R, R'und R" jeweils unabhängig voneinander ausgewählt sind aus $C_{1-6}$-Alkyl oder Phenyl, oder Carboxyamid-Polysiloxanen der allgemeinen Formel IVa

$$[R_a\text{-}\underset{\displaystyle B}{\overset{|}{Si}}O_{(3-a)/2}]_n\text{-}[\underset{\displaystyle R'_b}{\overset{|}{Si}}O_{(4-b)/2}]_m\text{-}[\underset{\displaystyle R''_2R'''}{\overset{|}{Si}}O_{1/2}]_k \qquad (IVa)$$

wobei R' und R" unabhängig voneinander ausgewählt sind aus $C_{1-6}$-Alkyl oder Phenyl, R und R''' unabhängig ausgewählt sind aus $C_{1-6}$Alkyl, $C_{1-6}$-Alkoxy, OH oder Phenyl, und die Zahl der Arten der durch

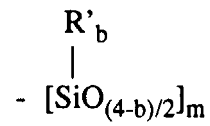

definierten strukturellen Einheiten mindestens 1 beträgt und a aus dem Bereich $0 \leq a \leq 2$ und b aus dem Bereich $1 \leq b \leq 3$ ausgewählt ist, und die durch die Variablen m, n, k festgelegte Zahl der Wiederholungen der Arten von strukturellen Einheiten aus den Bereichen

$1 \leq n \leq 100$, vorzugsweise $1 \leq n \leq 10$,
$10 \leq m \leq 800$, vorzugsweise $20 \leq m \leq 150$ und
$0 \leq k \leq \{(2-b)m + [(1-a)n + 2]\}$ ausgewählt ist,

sowie B einen organischen Rest der allgemeinen Formel V darstellt,

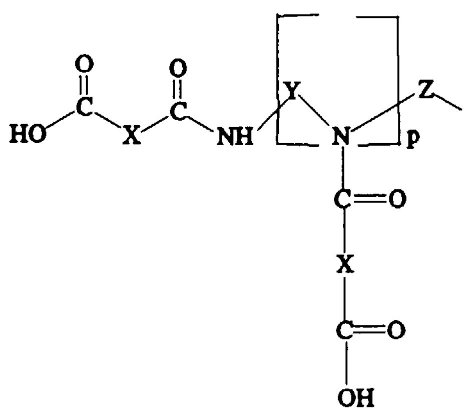

(V)

wobei p = 0 - 10, vorzugsweise 0 oder 1 und

Y und Z unabhängig voneinander jeweils einen divalenten Kohlenwasserstoffrest aus der Gruppe der aliphatischen Kohlenwasserstoffe oder einen divalenten Alkoxyalkylrest darstellen, und

X ein divalenter Kohlenwasserstoffrest aus der Gruppe von $-(CH_2)_y-$ mit $2 \leq y \leq 6$, vorzugsweise $2 \leq y \leq 4$, $-CHR^5-CH_2-$, $-CH_2-CHR^5-$ mit $R^5$ $C_{1-20}$-Alkyl oder $C_{2-20}$-Alkenyl, -CH=CH-, cyclischen oder bicyclischen, gesättigten oder ungesättigten Kohlenwasserstoffen oder der aromatischen Kohlenwasserstoffe darstellt, in kosmetischen Formulierungen, insbesondere in Haut-, Haar-, Nagelpflegemitteln oder Mundpflegemitteln gelöst.

Besonders bevorzugt sind die Carboxyamid-Polysiloxane der genannten Formel IV oder IVa, die einen Carboxylgruppengehalt von 0,02 bis 2,0 meq/g sowie eine Molmasse im Bereich von $1 \times 10^3$ bis $60 \times 10^3$ aufweisen,

Eine weitere bevorzugte Lösung der Aufgabe stellt die Verwendung der vorstehend genannten Carboxyamid-Polysiloxane dar, bei denen B einen organischen Rest der allgemeinen Formel V darstellt,

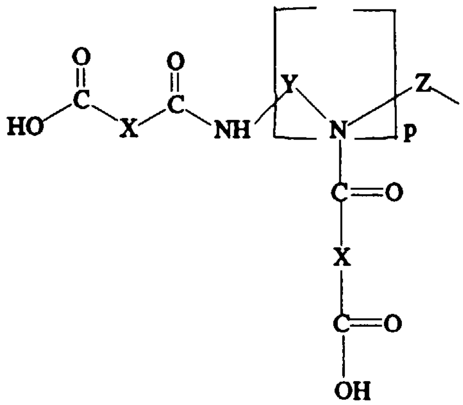

(V)

in dem p = 0 oder 1, X = $-CH_2CH_2-$, $-CH_2-CHR^5-$, $-CHR^5-CH_2-$ mit $R^5$ $C_{1-20}$-Alkyl oder $C_{2-20}$-Alkenyl, vorzugsweise $C_{6-18}$-Alkenyl, CH=CH- oder

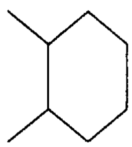
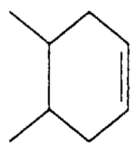
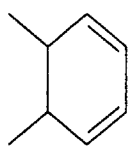
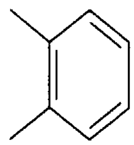
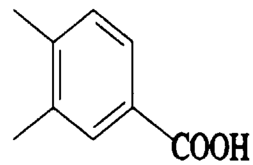

sind, Y = $-CH_2CH_2-$ und Z = $-CH_2CH_2CH_2-$ ist.

Die für die erfindungsgemäße Verwendung durch Umsetzung der aminofunktionellen Siloxane gemaß der allgemeinen Formel I

$$\begin{array}{ccc} A & R'_b & R''_3 \\ | & | & | \\ [R_a\text{-}SiO_{(3-a)/2}]_n & \text{-}[SiO_{(4-b)/2}]_m & \text{-}[SiO_{1/2}]_k \end{array} \qquad (I)$$

wobei R, R'und R" jeweils unabhängig voneinander ausgewählt sind aus $C_{1-6}$-Alkyl oder Phenyl, oder aminofunktionellen Polysiloxanen der allgemeinen Formel Ia

$$\begin{array}{ccc} A & R'_b & R''_2R''' \\ | & | & | \\ [R_a\text{-}SiO_{(3-a)/2}]_n & \text{-}[SiO_{(4-b)/2}]_m & \text{-}[SiO_{1/2}]_k \end{array} \qquad (Ia)$$

wobei R' und R" unabhängig voneinander ausgewählt sind aus $C_{1-6}$-Alkyl oder Phenyl, R und R"' unabhängig ausgewählt sind aus $C_{1-6}$-Alkyl, $C_{1-6}$-Alkoxy, OH oder Phenyl,und die Zahl der Arten der durch

$$-\,[\overset{\overset{R'_b}{|}}{Si}O_{(4-b)/2}]_m$$

definierten strukturellen Einheiten mindestens 1 beträgt, wobei a aus den Bereich $0 \leq a \leq 2$ und b aus dem Bereich $1 \leq b \leq 3$ ausgewählt ist, und die durch die Variablen m, n, k festgelegte Zähl der Wiederholungen der Arten von strukturellen Einheiten aus den Bereichen

$1 \leq n \leq 100$, vorzugsweise $1 \leq n \leq 10$,
$10 \leq m \leq 800$, vorzugsweise $20 \leq m \leq 150$ und
$O \leq k \leq \{(2\text{-}b)m + [(1\text{-}a)n + 2]\}$ ausgewählt ist,

sowie A einen organischen Rest der allgemeinen Formel II darstellt,

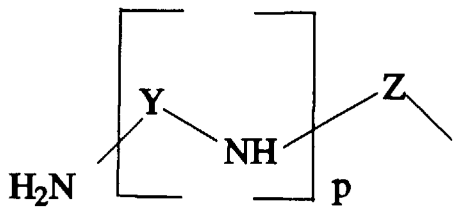

(II)

mit den Dicarbonsäureanhydriden der allgemeinen Formel III

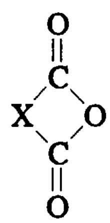

(III)

wobei X ein divalentes Kohlenwasserstoffradikal aus der Gruppe von $-(CH_2)_y$ mit $2 \leq y \leq 6$, $-CH_2\text{-}CHR^5\text{-}$ oder $-CHR^5\text{-}CH_2\text{-}$ mit $R^5$ $C_{1\text{-}20}$-Alkyl, vorzugsweise $C_{5\text{-}15}$-Alkyl oder $C_{2\text{-}20}$-Alkenyl, vorzugsweise $C_{6\text{-}18}$-Alkenyl, insbesondere Oct-2-enyl, Dec-2-enyl, Hexadec-2-enyl, Dodec-2-enyl, Octadec-2-enyl, -CH=CH-, cyclischen oder bicyclischen gesättigten oder ungesättigten Kohlenwasserstoffen oder der aromatischen Kohlenwasserstoffen darstellt, erhaltenen carboxyfunktionellen Siloxancopolymere bzw. Carboxyamid-Polysiloxane weisen eine Struktur gemäß der nachfolgend genannten Formel IV oder der vorstehend angegebenen Formel IVa auf:

$$[R_a\text{-}\overset{\overset{B}{|}}{Si}O_{(3-a)/2}]_n\text{-}[\overset{\overset{R'_b}{|}}{Si}O_{(4-b)/2}]_m\text{-}[\overset{\overset{R''_3}{|}}{Si}O_{1/2}]_k \qquad (IV)$$

wobei R, R', R" m, n, a, b und k die bereits genannte Bedeutung haben, sowie

B einen organischen Rest der nachfolgend angegebenen allgemeinen Formel V darstellt

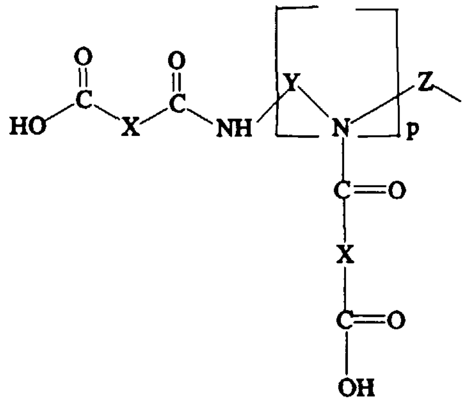

(V)

wobei p, X, Y und Z ebenfalls die vorstehend genannten Bedeutungen zeigen.

Bevorzugte aminofünktionalisierte Siloxane sind herstellbar aus den nachstehenden Verbindungen 1, 2 und 3:

$$A\text{-}SiR''_gR_{3-g} \quad (1)$$

$$\text{cyclischen } (SiR'_2\text{-}O\text{-})_h \quad (2a)$$

oder

$$\text{linearen } HO\text{-}(SiR'_2\text{-}O\text{-})_hH \text{ oder } R'_3Si\text{-}O\text{-}(SiR'_2\text{-}O\text{-})_hSiR'_3 \quad (2b)$$

$$R''_3Si\text{-}O\text{-}SiR''_3 \quad (3)$$

mit $g = 0 - 3$, d.h. 0, 1, 2 oder 3, vorzugsweise 0 oder 3
$h = 3 - 10$, vorzugsweise 3 - 5 in (2a)
$h = 3 - 10000$, vorzugsweise 10 - 200 in (2b)
und den vorstehend angegebenen Bedeutungen für A, R, R' und R"

Dabei sind R' und R" vorzugsweise $C_{1-3}$-Alkyl, insbesondere Methyl. R ist vorzugsweise $C_{1-3}$-Alkoxy, insbesondere Methoxy, Ethoxy oder OH.

Die Verbindungen werden unter basischen Bedingungen, vorzugsweise in Gegenwart von KOH umgesetzt.

Für den Fall g = 0, $R = OCH_3$, $R'=CH_3$, $R'' = CH_3$ ergeben sich im Siloxan folgende Grundbausteine:

$(CH_3)_3Si\text{-}O_{1/2}$, $(CH_3)_2Si(O_{1/2})_2$, $(CH_3)_2(CH_3O)Si\text{-}O_{1/2}$, $A\text{-}Si(OCH_3)_2(O_{1/2})$, $A\text{-}Si(OCH_3)(O_{1/2})_2$, $A\text{-}Si(O_{1/2})_3$.

Im so erhaltenen aminofünktionellen Siloxen beträgt die Aminzahl vorzugsweise 0,1 bis 0,5 meq/g. Das Molverhältnis von Verbindung (1) zu Verbindung (2)/h beträgt vorzugsweise 0,00375 bis 0,0375.

Anstelle der Verbindung (1) kann auch die entsprechende bereits amidierte Verbindung $B\text{-}SiR''_gR_{3-g}$ eingesetzt werden.

Verbindung (1) dient zur Funktionalisierung, Verbindung (2) zum Kettenaufbau und Verbindung (3) zur Terminierung.

Die eingesetzten aminofunktionellen Siloxane haben bevorzugterweise eine Viskosität im Bereich von 10 $mm^2/s$ bis ca. 10000 $mm^2/s$. Dies entspricht einer Molmasse im Bereich von $2 \times 10^3$ bis $60 \times 10^3$ g/Mol. Werden Aminosiloxane eingesetzt, deren Viskosität oberhalb des genannten Bereichs liegt, so haben die daraus erhaltenen Carboxyamid-Polysiloxane häufig keine zufriedenstellenden Eigenschaften. Insbesondere sind die Produkte dann häufig nicht homogen und/oder aufgrund zu hoher Viskosität nicht mehr rührbar. Wenn andererseits die Molmasse des eingesetzten Aminosiloxans den genannten Bereich unterschreitet, so ist die Haftung des hergestellten Carboxyamid-Polysiloxans an dem behandelten Material, insbesondere Haare, schlecht und/oder seine Verarbeitbarkeit nicht zufriedenstellend. Stark bevorzugt eingesetzte Aminosiloxane haben eine Viskosität im Bereich von 50 bis 500 $mm^2/s$.

Die erfindungsgemäß bevorzugt verwendeten Carboxyamid-Polysiloxane weisen einen Carboxylgruppengehalt

von 0,02 bis 2,0 meq/g auf, am stärksten bevorzugt jedoch 0,2 bis 0,5 meq/g.

Bevorzugt werden die Umsetzungsprodukte gemäß der vorstehend genannten Formel IV oder IVa zur Hydrophobierung als wäßrige stabile Emulsion eingesetzt, stärker bevorzugt als wäßrige Emulsionen mit einem Siloxangehalt von 3 - 90 Gew.-%, insbesondere 5 - 60 Gew.-%, besonders bevorzugt 7 - 40 Gew.-%.

Die Herstellung der wäßrigen Emulsionen erfolgt nach den in der DE-A 35 29 865, DE-A 38 00 629 und WO-A 95/22627 beschriebenen Verfahren, etwa indem das carboxyfunktionelle Siloxan mit Wasser und einem Amin oder Ammoniak oder Natronlauge oder Kalilauge (oder einer Kombination der Verbindungen) bei Temperaturen zwischen 0 - 100°C, bevorzugt bei 20 - 70°C, gut vermischt werden. Die Reihenfolge, in der die Komponenten zusammengefügt werden ist dabei gleichgültig. Die wäßrige Mischung wird anschließend in einem geeigneten Homogenisator (z.B. Spalthomogenisator) zu einer Emulsion verarbeitet. Vor der Emulgierung können zusätzlich die weiter unten aufgeführten Öle und Emulgatoren zugefügt werden. Diese werden bevorzugt vor der Homogenisierung eingemischt.

Als weiterer Zusatz können Öl- oder Fettsäuren in einer Konzentration zwischen 0,1 - 5 Gew.-% zu der Emulsion zugesetzt werden.

Insbesondere handelt es sich bei den Siloxanen der allgemeinen Formeln I oder Ia und IV oder IVa um lineare Siloxane mit terminalen organofunktionellen Gruppen, bei denen n = 2, a = 2, b = 2, R = R' = R'' = $CH_3$, R''' = $OCH_3$ oder OH, Y = $-CH_2CH_2-$, Z = $-CH_2CH_2CH_2-$ und p = 0 oder 1 ist, wobei die anderen Substituenten und Variablen die bereits genannte Bedeutung haben. Besonders bevorzugt sind unter den Siloxancopolymeren der allgemeinen Formeln I oder Ia und IV oder IVa lineare Siloxane mit terminalen Trimethoxysilylresten und seitlichen organofunktionellen Gruppen, bei denen n = 1 - 5, m = 10 - 100, a = 1, b = 2, R = R' = R'' = $CH_3$, R''' = $OCH_3$ oder OH, Y = $-CH_2CH_2-$, Z = $-CH_2CH_2CH_2-$ und p = 0 oder 1 ist und die anderen Substituenten und Variablen die genannte Bedeutung haben. Am stärksten bevorzugt ist p = 0. Vor dem Emulgieren ist R''' in der Regel $OCH_3$, beim Kontakt mit Wasser erfolgt zumindest teilweise Hydrolyse zu OH.

Die Siloxane der allgemeinen Formel I oder Ia bzw. IV oder IVa besitzen vorzugsweise Molmassen im Bereich zwischen 3000 und 10000. Es kommen entweder lineare, verzweigte oder cyclische Siloxane in Betracht, wobei die linearen oder verzweigten Siloxane Silanol-, Alkoxy-, Kohlenwasserstoff- oder Triorganosiloxyendgruppen aufweisen können. Bei einer bevorzugten Variante werden lineare oder verzweigte Siloxane zur Hydrophobierung verwendet, die Endgruppen der folgenden Formel VI

$$\overset{\displaystyle B}{\overset{|}{[R_a\text{-}SiO_{(3-a)/2}]}} \qquad (VI)$$

mit a = 2 aufweisen.

Ferner können die in Formeln I oder Ia und IV oder IVa beschriebenen Siloxancopolymere verschiedene Siloxyeinheiten besitzen, die durch unterschiedliche Quotienten der Variablen a/n und/oder b/m gekennzeichnet sind. So kann ein Siloxancopolymer z.B. lineare Einheiten

$$\text{-}\ \overset{\displaystyle R'_b}{\overset{|}{[SiO_{(4-b)/2}]}}\ \text{-}$$

enthalten, bei denen b = 2 ist. Zugleich weist es Verzweigungspunkte auf, bei denen b = 1 bedeutet und Endgruppen enthalten sind, bei denen b = 3 ist.

Das gleiche gilt für die wiedergegebenen Siloxaneinheiten der folgenden Struktur:

$$\overset{\displaystyle B}{\overset{|}{[R_a\text{-}SiO_{(3-a)/2}]}}$$

Als Reste R, R' oder R" kommen bevorzugt Methyl-, Phenyl- oder Ethylreste in Betracht, besonders bevorzugt ist R = R' = Me.

Bei den Resten X handelt es sich bevorzugt um folgende divalente Kohlenwasserstoffreste: -CH=CH- oder $-CH_2-CH_2-$, $-C(R^1R^2)-CH_2-$, wobei $R^1$ oder $R^2$ = $-(CH{=}CH)_d(CR^3R^4)_k-Q$, mit $0 \leq d \leq 2$, $0 \leq k \leq 30$; Q ausgewählt aus H, COOH; $R^3$ oder $R^4$ gleich H oder $CH_3$ oder

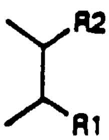 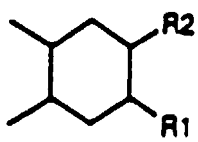 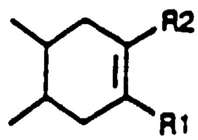 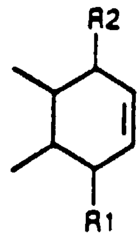 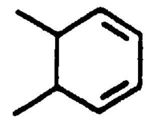

wobei $R^1$, $R^2$ unabhängig voneinander sind und H, $C_1$ - $C_{30}$-Alkyl (linear oder verzweigt), z.B. Methyl, Ethyl usw., oder $C_2$ - $C_{30}$-Alkenyl (linear oder verzweigt), z.B. Allyl, Hexenyl, Dodecenyl usw., oder Phenyl oder substituerte Arylverbindungen sind, wobei die Substituenten bevorzugt Chlor, Alkoxy oder Acyloxy sind;

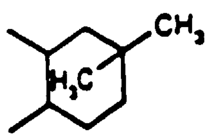 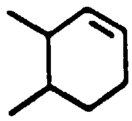 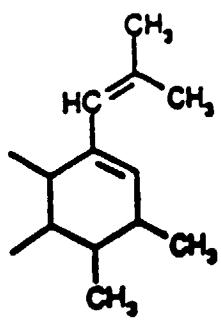 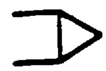

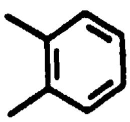 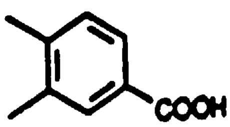 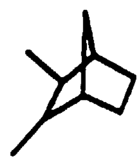

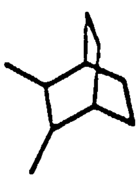 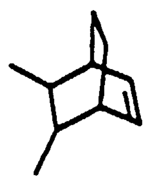 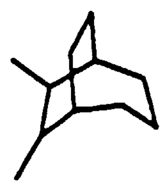 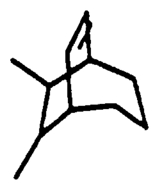

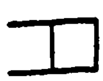 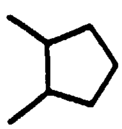

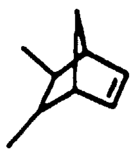

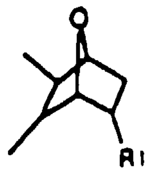

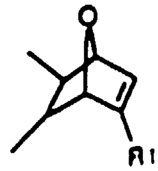

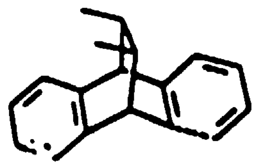

Bei den Resten Y und Z handelt es sich bevorzugt um divalente Kohlenwasserstoffreste wie Ethylen, Dimethylen, Hexamethylen, Octaethylen und/oder um Sauerstoff enthaltende divalente Kohlenwasserstoffe wie Ethylenoxid, Trimethylenoxid oder Oligomere oder Polymere von diesen Verbindungen. Besonders bevorzugt ist X = $-CH_2-CH_2-$ oder $-CH_2-CHR^5-$ oder $-CHR^5-CH_2-$mit $R^5$ $C_{6-18}$-Alkenyl und Z = $-CH_2-CH_2-CH_2-$. Die Variable p kann Werte zwischen 0 und 10 annehmen, bevorzugt aber ist p = 0 oder 1. Am stärksten bevorzugt ist p = 0 und Z = $-CH_2-CH_2-CH_2-$.

Eine bevorzugte Verbindung ist $(CH_3)_3Si-(O-Si(CH_3)_2)m_n-(O-SiACH_3)_n-OSi-(CH_3)_3$ mit A = $CH_2-CH_2-CH_2-NH-CH_2-CH_2-NH_2$, besonders bevorzugt mit einer Viskosität von etwa 200 $mm^2/s$ und einer Aminzahl von 0,27. Die Umsetzung der Aminogruppen erfolgt vorzugsweise mit Octenylbernsteinsäureanhydrid.

Man erhält eine Verbindung (IV):

$$(CH_3)_3Si-(O-Si(CH_3)_2)_m-(O-SiBCH_3)_n-OSi(CH_3)_3$$

mit B:

$$CH_2-CH_2-CH_2-\underset{\displaystyle R^x}{\underset{|}{N}}-CH_2-CH_2-NH-CO-\underset{\displaystyle CH_2-CH=CH-(CH_2-)_4CH_3}{\underset{|}{C}}H-CH_2-COOH$$

mit $R^x$:

$$C(O)-CH(CH_2-CH=CH-(CH_2-)_4CH_3)-CH_2-COOH$$

Die Carboxylgruppen der Verbindungen gemäß Formeln IV oder IVa können sowohl als freie Carbonsäuren als auch in partieller oder vollständiger Salzform, beispielweise als Alkalimetallsalze wie Natrium- oder Kaliumsalze, als Ammoniumsalze oder als Alkylammoniumsalze vorliegen. Besonders bevorzugt zur Salzbildung sind primäre oder tertiäre Amine, insbesondere 2-Amino-2-methyl-propanol-1, Ethanolamin, Triethanolamin Polyethylenglykolamine, Methylpolyethylenglykolamine und/oder Morpholin. Im allgemeinen werden sie in ihrer Salzform eingesetzt.

Die beschriebenen Polysiloxan-Emulsionen enthalten üblicherweise 3 bis 90 Gew.-%, insbesondere 5 bis 60 Gew.-%, besonders bevorzugt bis zu 40 Gew.-% carboxylfunktionalisierte Polysiloxane gemäß Formel IV. Die Emulsionen werden normalerweise in Mengen von 0,1 bis 20 Gew.-%, insbesondere 0,5 bis 15 Gew.-%, eingesetzt.

Bevorzugt verwendet werden die Carboxyamid-Polysiloxane als Haarfestiger, als Conditioner, als Verdickungsmittel sowie als Hilfsmittel bei der Haarfärbung, dem Haarbleichen und beim Dauerwellverfahren. Besonders vorteilhaft werden die Carboxyamid-Polysiloxane in Form von Emulsionen eingesetzt. Diese Emulsionen können alle in der Kosmetik üblichen Wirk- und Hilfsstoffe enthalten.

Üblicherweise werden emulgierende Substanzen mit Wasser, Ölen, Fetten und ggf. Fettalkoholen, Fettsäuren, Konservierungsmitteln, Polymeren, Konsistenzgebern und gelbildenden Stoffen zusammengebracht

Als Wirkstoffe können Lichtschutzmittel, Bleichmittel, Haarfärbemittel, Dauerwellenmittel, kationische Tenside, anionische Tenside, Feuchthaltemittel, Harnstoff, Vitamine, Panthenol, Panthenolethylether und/oder Bisabolol enthalten sein.

Bei den erfindungsgemäßen Verwendungen können zusätzlich zu den Carboxyamid-Polysiloxanen auch alle normalerweise in kosmetischen Mitteln verwendeten Bestandteile eingesetzt werden, insbesondere ionische und nichtionische oberflächenaktive Mittel, Schaumsynergisten, Schaumstabilisatoren, opakmachende Mittel, Sequestriermittel, Verdickungsmittel, Emulgatoren, weichmachende Mittel, Konservierungsmittel, Proteinderivate, natürliche Substanzen, Farbstoffe, Parfüms und ggf. weitere Polymere oder Hilfsstoffe.

Trägt man die erfindungsgemäßen Polymere als wäßrige, wäßrig alkoholische oder alkoholische Mittel bzw. in kosmetischen Emulsionen auf die Haare auf, dann lassen sich diese leichter entwirren und besitzen eine deutlich verbesserte Naßkämmbarkeit als das bei Verwendung der oben genannten siliconhaltigen Derivate oder quaternären Polyammonium-Polymeren mit Kohlenwasserstoffketten der Fall ist. Die Haare werden weniger elektrostatisch aufgeladen, was das Frisieren erleichtert. Die Haare befinden sich in einem sehr guten kosmetischen Zustand; auffallend ist der extrem gute angenehme Griff des trockenen Haares. Am deutlichsten zeigen sich die Vorteile in einer kosmetischen Emulsion (siehe Beispiele).

Trägt man die erfindungsgemäßen Polymere als wäßrige, wäßrig alkoholische oder alkoholische Mittel bzw. in kosmetischen Emulsionen auf die Haut auf, dann läßt sich ein angenehmes Hautgefühl erzeugen. Das Hautgefühl ist besser als mit den oben genannten siliconhaltigen Derivate oder mit quaternären Polyammonium-Polymeren mit Kohlenwasserstoffketten.

Die Erfindung wird nachfolgend an Beispielen näher erläutert.

HERSTELLUNGSBEISPIEL 1

Insgesamt 300 g eines Polydimethylsiloxans mit terminalen Aminopropylresten (Aminzahl: 0,46 meq/g) wird unter Schutzgas in einem 3-Halskolben, der mit Rührer und Innenthermometer ausgerüstet ist, vorgelegt und auf dem Wasserbad auf 70°C erwärmt. Bernsteinsäureanhydrid (13,9 g) werden in einer Laboranalysenmühle A10 gemahlen und über einen Pulvertrichter langsam zu dem Siloxan dosiert. Die Mischung wird anschließend 4 Stunden bei 70°C langsam gerührt und danach auf 50°C abgekühlt.

IR-spektroskopisch wurde die vollständige Umwandlung von Aminosiloxan und Bernsteinsäureanhydrid in ein carboxyfunktionelles Siloxan der unten gezeigten Struktur nachgewiesen. (Die Anhydridbande bei ca. 1780 $cm^{-1}$ verschwindet, zwei neue Carbonylbanden, 1620 $cm^{-1}$ und 1720 $cm^{-1}$, entstehen, COOH-Valenzschwingung zwischen 2400 - 3500 $cm^{-1}$) Die erfolgreiche Umsetzung konnte auch mittels $^{13}C$-NMR nachgewiesen werden.

Zu dem oben beschriebenen Reaktionsprodukt werden 1200 g entionisiertes Wasser und 8,5 g Ethanolamin zugegeben und bei 50°C 10 min mit em Ultraturrax (Euro Turrax T20b von IKA) emulgiert. Anschließend wird diese Voremulsion noch in einem Spalthomogenisator zu einer lagerstabilen Emulsion verarbeitet.

Struktur des Umsetzungsproduktes:

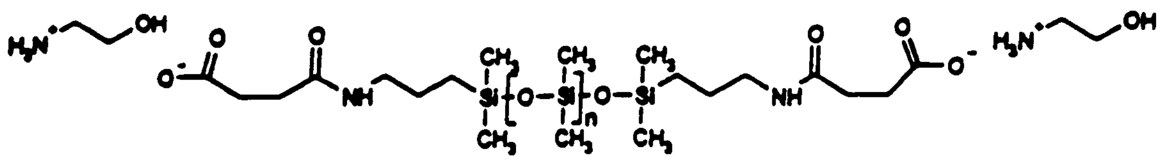

n ~ 55

HERSTELLUNGSBEISPIEL 2

Die Herstellung einer Siliconölemulsion erfolgt gemaß Beispiel 1. Statt Ethanolamin werden jedoch 12,4 g 2-Amino-2-methylpropanol zur Neutralisation eingesetzt.

Struktur des Umsetzungsproduktes:

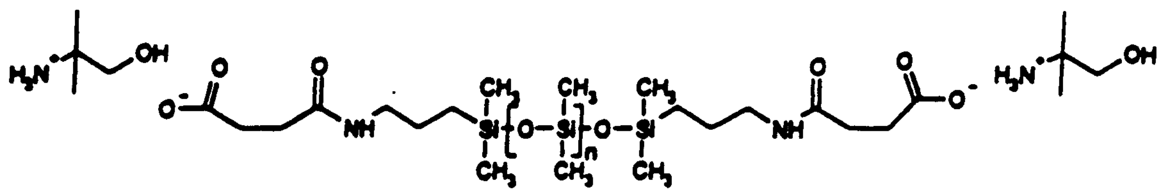

n ~ 55

HERSTELLUNGSBEISPIEL 3

Herstellungsbeispiel 2 wurde wiederholt, wobei jedoch ein pH-Wert von 6 bis 7 eingehalten wurde.

**PRÜFUNGSBEISPIELE**

**Prüfungen:**

**Naßkämmbarkeit:** subjektive Beurteilung von ausgebildetem erfahrenem Friseur- und Laborpersonal an Haarsträhnen
Notenskala: 1 (sehr gut), 2 (gut) und 3 (schlecht)
Doppelbestimmung von zwei Personen an gebleichten Haarsträhnen mit mitteleuropäischem Haar; Breite 6 cm; Länge 25 cm.

**Kämmkraftabnahme:** Prüfung der Naßkämmbarkeit an einer Zug/DruckPrüfmaschine; Messung der Abnahme der Kämmkraft im Vergleich zu einer unbehandelten Haarsträhne; Mittelwerte aus 15 Einzelmessungen; Haare wie oben

**statische Aufladung:** subjektive Beurteilung von ausgebildetem erfahrenem Friseur- und Laborpersonal nach 10 maligem Kämmen der oben genannten Haarsträhnen im trockenen Zustand im Klimaraum bei 50% relativer Feuchte.
Notenskala: 1 (keine), 2 (schwach), 3 (stark)

**Griff** des trockenen Haares: subjektive Beurteilung von ausgebildetem erfahrenen Friseur- und Laborpersonal
Notenskala: 1 (sehr zart), 2 (zart), 3 (rauh)

**Herstellung der Prüfemulsionen**

| **Emulsion 1** | | |
|---|---|---|
| | g | |
| Phase A: | 1,00 | Siliconderivat (Wirkstoff) |
| | 1,50 | Ceteareth-25 |
| | 1,50 | Ceteareth-6 und Stearylalkohol |
| | 6,00 | Cetearyl-octanoat |
| | 3,00 | Cetearylalkohol |

(fortgesetzt)

| Emulsion 1 | | |
|---|---|---|
| | g | |
| Phase B: | 2,00 | Propylenglykol |
| | 0,30 | Imidazolidinyl-Harnstoff |
| | 0,10 | Benzylalkohol, Methylchloroisothiazolinon, Methylisothiazolinon |
| | ad 100.00 g | Wasser |

| Emulsion 2 | | |
|---|---|---|
| | g | |
| Phase A: | 1,50 | Ceteareth-25 |
| | 1,50 | Ceteareth-6 und Stearylalkohol |
| | 6,00 | Cetearyl-octanoat |
| | 3,00 | Cetearylalkohol |
| Phase B: | 1,00 | Siliconderivat (Wirkstoff) |
| | 2,00 | Propylenglycol |
| | 0,30 | Imidazolidinyl-Harnstoff |
| | 0,10 | Benzylalkohol, Methylchloroisothiazolinon, Methylisothiazolinon |
| | ad 100,00 g | Wasser |

Phase A und B wurden getrennt unter Rühren auf 80°C erhitzt. Phase A wurde unter Rühren in Phase B gegeben und abgekühlt.

Der pH-Wert wurde nach der Herstellung mit Citronensäure eingestellt.

## Tabelle 1

Ergebnisse der anwendungstechnischen Prüfungen

| Polymer Nr. | Emulsion Nr. | pH-Wert | Naßkämmbarkeit (Note) | Kämmkraft-abnahme % | statische Aufla-dung | Griff (Note) |
|---|---|---|---|---|---|---|
| Herstellungsbsp. 1 | 2 | 2-3 | 1-2 | 69 | 1 | 1-2 |
| Herstellungsbsp. 2 | 2 | 2-3 | 1 | 72 | 1 | 1 |
| Herstellungsbsp. 3 | 2 | 6-7 | 1 | 75 | 1 | 1 |
| Vergleichsbeispiele | | | | | | |
| ohne Polymer | ohne Polymer | 2-3 | 2-3 | 0 | 3 | 3 |
| Quaternium-80 | 1 | 2-3 | 2-3 | 25 | 3 | 3 |
| Dimethicon-Copolymer | 1 | 2-3 | 2-3 | 16 | 3 | 3 |
| Polyquaternium-11 | 2 | 2-3 | 2 | 36 | 3 | 2 |
| Dimethicon-Propyl-PG-Betain | 1 | 2-3 | 3 | 11 | 3 | 3 |
| Dimethicon-Propyl-PG-Betain | 2 | 2-3 | 3 | 17 | 3 | 3 |

## Patentansprüche

1. Verwendung von Carboxyamid-Polysiloxanen der allgemeinen Formel IV

$$[R_a\text{-}\overset{B}{Si}O_{(3-a)/2}]_n\text{-}[\overset{R'_b}{Si}O_{(4-b)/2}]_m\text{-}[\overset{R''_3}{Si}O_{1/2}]_k \qquad \text{(IV)}$$

wobei R, R'und R" jeweils unabhängig voneinander ausgewählt sind aus $C_{1\text{-}6}$-Alkyl oder Phenyl, oder Carboxyamid-Polysiloxanen der allgemeinen Formel IVa

$$[R_a\text{-}\overset{B}{Si}O_{(3-a)/2}]_n\text{-}[\overset{R'_b}{Si}O_{(4-b)/2}]_m\text{-}[\overset{R''_2R'''}{Si}O_{1/2}]_k \qquad \text{(IVa)}$$

wobei R' und R" unabhängig voneinander ausgewählt sind aus $C_{1\text{-}6}$-Alkyl oder Phenyl, R und R'" unabhängig ausgewählt sind aus $C_{1\text{-}6}$-Alkyl, $C_{1\text{-}6}$-Alkoxy, OH oder Phenyl, und die Zahl der Arten der durch

$$-\ [\overset{R'_b}{Si}O_{(4-b)/2}]_m$$

definierten strukturellen Einheiten mindestens 1 beträgt und a aus dem Bereich $0 \leq a \leq 2$ und b aus dem Bereich $1 \leq b \leq 3$ ausgewählt ist, und die durch die Variablen m, n, k festgelegte Zahl der Wiederholungen der Arten von strukturellen Einheiten aus den Bereichen

$1 \leq n \leq 100$
$10 \leq m \leq 800$ und
$0 \leq k \leq \{(2\text{-}b)m + [(1\text{-}a)n + 2]\}$ ausgewählt ist,

sowie B einen organischen Rest der allgemeinen Formel V darstellt,

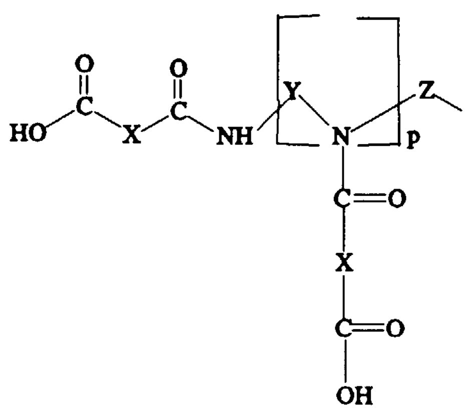

(V)

wobei p = 0 - 10 und

Y und Z unabhängig voneinander jeweils einen divalenten Kohlenwasserstoffrest aus der Gruppe der aliphatischen Kohlenwasserstoffe oder einen divalenten Alkoxyalkylrest darstellen, und

X ein divalenter Kohlenwasserstoffrest aus der Gruppe von $-(CH_2)_y-$ mit $2 \leq y \leq 6$, $-CH_2-CHR^5-$ oder $-CHR^5-CH_2-$ mit $R^5$ $C_{1-20}$-Alkyl oder $C_{2-20}$-Alkenyl, -CH=CH-, cyclischen oder bicyclischen, gesättigten oder ungesättigten Kohlenwasserstoffen oder der aromatischen Kohlenwasserstoffe darstellt, in kosmetischen Formulierungen.

2. Verwendung nach Anspruch 1, wobei die Carboxyamid-Polysiloxane der genannten Formel IV oder IVa einen Carboxylgruppengehalt von 0,02 bis 2,0 mMol/g sowie eine Molmasse im Bereich von $1 \times 10^3$ bis $60 \times 10^3$ aufweisen.

3. Verwendung nach Anspruch 1 oder 2, wobei B einen organischen Rest der allgemeinen Formel V darstellt,

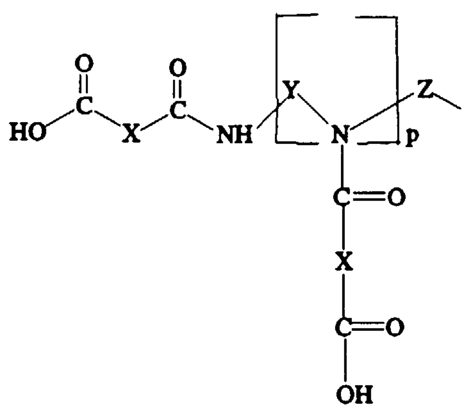

(V)

in dem p = O oder 1, X = $-CH_2CH_2-$, $-CH_2-CHR^5-$ oder $CHR^5-CH_2-$ mit $R^5$ $C_{1-20}$-Alkyl oder $C_{2-20}$-Alkenyl, -CH=CH- oder

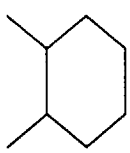
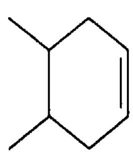
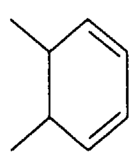
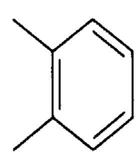
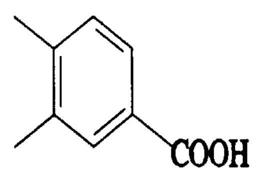

sind, Y = $-CH_2CH_2-$ und Z = $-CH_2CH_2CH_2-$ ist.

4. Verwendung nach einem der Ansprüche 1 bis 3, wobei das Carboxyamid-Polysiloxan einen Carboxylgruppengehalt von 0,2 bis 0,5 mMol/g aufweist und die Mohnasse im Bereich von 3000 bis 10000 liegt.

5. Verwendung nach einem der Ansprüche 1 bis 4, wobei X $-CH_2-CHR^5-$ oder $-CHR^5-CH_2-$ mit $R^5$ $C_{6-18}$-Alkenyl, -CH =CH- oder $-CH_2-CH_2-$ ist.

6. Verwendung nach einem der Ansprüche 1 bis 5, wobei die nichtamidierten Carboxylgruppen der Carboxyamid-Polysiloxane zumindest teilweise in neutralisierter Form vorliegen.

7. Verwendung nach einem der Ansprüche 1 bis 6 als Haut- und Haarpflegemittel.

8. Verwendung nach einem der Ansprüche 1 bis 7 in Form von Emulsionen.

9. Verwendung nach einem der Ansprüche 1 bis 8 in Shampoos, Haarfestigern, als Hilfsmittel vor, während oder nach der Haarfärbung, dem Haarbleichen oder dem Dauerwellverfahren, zur Mundpflege oder zur Finger- und Fußnagelpflege.

**10.** Mittel zur Haut- und Haarpflege in Form einer wäßrigen Emulsion, enthaltend

1 bis 90 Gew.- % eines Carboxyamid-Polysiloxans, wie es in einem der Ansprüche 1 bis 6 definiert ist,
0 bis 30 Gew.-% Emulgator,
0 bis 50 Gew.-% Paraffin, Paraffinöl, Mineralöle, natürliche Fette oder Öle oder synthetische oder natürliche Wachse.